Europäisches Patentamt

European Patent Office

Office européen des brevets

(19)

(11)  EP 0 509 213 B1

(12)  **EUROPÄISCHE PATENTSCHRIFT**

(45) Veröffentlichungstag und Bekanntmachung des Hinweises auf die Patenterteilung:
**10.04.1996  Patentblatt 1996/15**

(51) Int. Cl.⁶: **C07F 7/18**, C07C 51/60

(21) Anmeldenummer: 92102811.4

(22) Anmeldetag: **20.02.1992**

(54) **Verfahren zur gleichzeitigen und kontinuierlichen Herstellung von Carbonoyloxysilanen und Carbonsäurechloriden**

Process for the simultaneous and continuous production of carbonyloxy silanes and of carboxylic acid chlorides

Procédé pour la préparation simultanée et continue de carbonoyloxysilanes et de chlorures d'acides

(84) Benannte Vertragsstaaten:
**BE DE ES FR GB IT NL**

(30) Priorität: **18.04.1991 DE 4112650**

(43) Veröffentlichungstag der Anmeldung:
**21.10.1992  Patentblatt 1992/43**

(73) Patentinhaber: **HÜLS AKTIENGESELLSCHAFT**
**D-45764 Marl (DE)**

(72) Erfinder:
• **Seiler, Claus-Dietrich, Dr.**
 **W-7888 Rheinfelden (DE)**
• **Schork, Reinhold, Dr.**
 **W-7888 Rheinfelden (DE)**
• **Frings, Albert, Dr.**
 **W-7888 Rheinfelden (DE)**
• **Kötzsch, Hans-Joachim, Dr.**
 **W-7888 Rheinfelden (DE)**
• **Rauleder, Hartwig, Dr.**
 **W-7888 Rheinfelden (DE)**

(56) Entgegenhaltungen:
EP-A- 0 003 317          DE-B- 1 257 432
FR-A- 1 003 073          US-A- 3 974 198
US-A- 4 329 484          US-A- 4 332 956
US-A- 4 556 725

**Beschreibung**

Die vorliegende Erfindung betrifft ein Verfahren zur gleichzeitigen und kontinuierlichen Herstellung von Carbonoyloxysilanen und Carbonsäurechloriden durch Umsetzung von Organo-Chlorsilanen mit Monocarbonsäureanhydriden.

Carbonoyloxysilane haben vielseitige Anwendung in der chemischen Industrie gefunden. So eignen sie sich z. B. als vernetzende Siliciumverbindungen bei der Herstellung unter Ausschluß von Wasser lagerfähiger und bei Zutritt von Feuchtigkeit bei Raumtemperatur zu Elastomeren härtbarer Massen. Solche Massen werden durch Vermischen von kondensationsfähige Endgruppen enthaltenden Diorganopolysiloxanen und vernetzenden Siliciumverbindungen erhalten. Als Beispiele für dazu geeignete Carbonoyloxysilane seien die Verbindungen Vinyl-, Methyl- und Ethyltriacetoxysilan genannt. Carbonsäurechloride sind wichtige, zum Teil wertvolle Rohstoffe, die vor allem zur Synthese organischer Verbindungen eingesetzt werden.

Es ist bekannt, Alkanoyloxysilane kontinuierlich durch Umsetzung von Organo-Chlorsilanen mit Alkansäuren bei erhöhter Temperatur in einer Kolonne herzustellen (DE-PS 28 01 780), wobei die Alkansäure in verdampfter Form von unten nach oben dem Organo-Chlorsilan entgegengeführt wird und mit diesem unter Bildung von Alkanoyloxysilan und Chlorwasserstoff zur Umsetzung gelangt.

Nachteilig bei dieser Verfahrensweise ist, daß der gebildete Chlorwasserstoff über längere Zeiträume mit den eingesetzten Alkansäuren unter Bedingungen in Berührung ist, bei denen die Bildung von Wasser und Alkanoylchloriden erfolgt, was einerseits zusätzliche Siloxanbildung zur Folge hat, andererseits aufwendige Tiefkühlung am Kolonnenkopf erfordert, um die im Chlorwasserstoffstrom entsprechend ihrem Dampfdruck mitgeführten Stoffe abzuscheiden und den Chlorwasserstoff für Zwecke, bei denen besondere Reinheitsanforderungen gestellt werden, einsetzbar zu machen. Bei dieser Verfahrensweise ist auch von Nachteil, daß die Dosierung der in die Kolonne eingeführten Alkansäuren durch eine aufwendige Vorrichtung geregelt werden muß, die von einer Temperaturmeßstelle im unteren Teil der Kolonne gesteuert wird.

Von Nachteil ist bei der Verfahrensweise der DE-PS 28 01 780 auch noch, daß die mit der Freisetzung großer Gasmengen verbundene Reaktion unter Vakuum durchgeführt werden muß, was besondere Druckstabilisierungseinrichtungen erforderlich macht.

Aufgrund der mit der Verfahrensweise gemäß DE-PS 28 01 780 verbundenen Mängel fällt in der Kolonnenblase z. B. Ethyltriacetoxysilan nur mit einem Ethyltriacetoxysilan-Gehalt von lediglich 94 %, bei einem Gehalt von 2 % Essigsäure und offensichtlich 4 % Siloxanen sowie einem Gehalt an hydrolysierbarem Chlor von ca. 50 ppm, an.

Eine nur geringfügige Verbesserung dieser Situation wird erreicht, wenn bei dieser bekannten Verfahrensweise die Zugabe der Alkansäure zusammen mit dem Organo-Chlorsilan im unteren Teil der Kolonne und im oberen Teil der Kolonne die Aufgabe von Alkansäure erfolgt (DE-PS 32 21 702).

Es ist auch bekannt, die Umsetzung von Organo-Chlorsilanen mit den den Alkansäuren entsprechenden in Gegenwart von Pyridin durchzuführen (DE-A 1257432); die gleichzeitige Herstellung von Carbonoyloxysilanen und Carbonsäurechloriden durch Umsetzung von Organo-Chlorsilanen mit Monocarbonsäureanhydriden ist aus der FR-A-1003073 bekannt. Allerdings ist bei diesen Umsetzungen nur eine diskontinuierliche Verfahrensweise beschrieben worden.

Es ergab sich deshalb das Problem, ein kontinuierliches, großtechnisch anwendbares Verfahren zur Herstellung von Carbonoyloxysilanen zu finden, das die Mängel der Verfahrensweisen gemäß dem genannten Stand der Technik nicht aufweist und bei dem anstelle des relativ wertlosen Nebenproduktes Chlorwasserstoff hochwertiges Nebenprodukt in Form von Carbonsäurechlorid anfällt.

Das Problem wurde durch die Entwicklung eines Verfahrens zur gleichzeitigen und kontinuierlichen Herstellung von Carbonoyloxysilanen und Carbonsäurechloriden durch Umsetzung von Organo-Chlorsilanen mit überschüssigem Monocarbonsäureanhydrid bei erhöhter Temperatur, Abtrennung des Carbonsäurechlorids nach erfolgter Umsetzung und daran anschließender Gewinnung des Carbonoyloxysilans gelöst, das dadurch gekennzeichnet ist, daß man die Ausgangskomponenten unter Zusatz von im Reaktionsgemisch löslichen organischen Basen, deren Salzen oder organischen Säureamiden bei Temperaturen von 25 bis 100 $^\circ$C durch einen Reaktor oder mehrere Reaktoren leitet, anschließend in einem Destillationsreaktor das entstandene Carbonsäurechlorid im Vakuum entfernt und das nach Abtrennung des Carbonsäurechlorids verbleibende Reaktionsgemisch in den Mitteleinlauf einer Kolonne überführt, unter Vakuum am Kolonnenkopf überschüssiges Carbonsäureanhydrid abdestilliert und das Carbonoyloxysilan aus der Kolonnenblase abzieht.

Die nach dem erfindungsgemäßen Verfahren als Ausgangsprodukte einsetzbaren Chlorsilane entsprechen der allgemeinen Formel

$$R^1_a \, R^2_b \, SiCl_{4-a-b,}$$

wobei a den Wert 3, 2 oder 1 und b den Wert 1 oder 0 haben kann.

Bevorzugt ist a = 1 und b = 0.

$R^1$ und $R^2$ stehen für Wasserstoff bzw. gesättigte gleiche oder ungleiche oder ungesättigte Kohlenwasserstoff-Reste mit jeweils 1 bis 10 Kohlenstoffatomen, die ggf. funktionelle Gruppen, z. B. Halogen, besitzen können, die unter den gegebenen Reaktionsbedingungen nicht angegriffen werden.

So können als siliciumhaltige Ausgangsprodukte beispielsweise folgende Verbindungen eingesetzt werden:

Vinyltrichlorsilan,

Ethyltrichlorsilan,

Methyltrichlorsilan,

Trichlorsilan,

Propyltrichlorsilan,

2-Chlorethyl-methyldichlorsilan.

Die erfindungsgemäß eingesetzten Carbonsäureanhydride leiten sich hauptsächlich von einbasischen aliphatischen Säuren ab, vorzugsweise von solchen mit 2 bis 4 Kohlenstoffatomen, wobei die Kohlenwasserstoffreste der Säure auch ungesättigt sein können. Als Beispiele für derartige Anhydride seien Ethausäureanhydrid, Propansäureanhydrid und Butansäureanhydrid genannt. Aber auch Verbindungen, bei denen ein Wasserstoffatom des aliphatischen Restes durch einen Phenylrest ersetzt ist, wie z. B. Phenylmethansäure-Anhydrid, sind einsetzbar.

Bei dem erfindungsgemäßen Verfahren wird ein Reaktorsystem benutzt, das im einfachsten Fall aus zwei hintereinandergeschalteten Reaktionsgefäßen besteht, durch welche die Ansgangsprodukte strömen und in denen ihre Verweilzeit durch entsprechende Gefäßauslegung den produktspezifischen Reaktionserfordernissen angepaßt werden kann. Die Verbindung der Reaktoren erfolgt zweckmäßigerweise in der Form, daß das gebildete Reaktionsgemisch dem vorangehenden Reaktor kopfseitig entnommen und dem nachfolgenden Reaktor bodenseitig zugeführt wird. Der zweite Reaktor wird als Destillationsreaktor bezeichnet und ist mit einem mit Rückflußteiler und Kühler ausgestatteten Kolonnenkopf versehen, über den unter einem Druck von 5 bis 300 mbar die Entfernung der in dem vorangehenden Reaktor gebildeten und sich in dem Destillationsreaktor im Verlauf der destillativen Entfernung der Carbonsäurechloride noch bildenden Carbonsäurechloride erfolgt. Die Anzahl der Reaktoren, in denen die Umsetzung und Einstellung des Reaktionsgleichgewichtes erfolgt, kann beliebig gewählt werden. Meistens genügt ein Reaktor, um zu gewährleisten, daß die vollständige Umsetzung der unterschüssig angewandten Einsatzkomponente erfolgt und somit das den Destillationsreaktor verlassende, in den Mitteleinlauf einer Kolonne eingeleitete Reaktionsgemisch die Unterschußkomponente nicht mehr enthält. Als Reaktoren im Sinne der vorliegenden Erfindung eignen sich vor allem Röhrenreaktoren.

Die Reaktoren sind beheizbar, z. B. über Doppelmantelsysteme. Dabei ist zu gewahrleisten, daß die Reaktoren unterschiedlichen Temperaturbereichen ausgesetzt werden können, um für die jeweiligen Ausgangsstoffe die günstigsten Reaktionstemperaturen zur Erzielung optimaler Umsetzungsraten einzustellen. Die Durchwirbelung der Reaktionsmischung ist im Destillationsreaktor besonders wirksam zu gestalten, z. B. durch Einsatz intensiv wirkender Rührsysteme, um damit die möglichst vollständige Entfernung der entsprechend den eingesetzten Ausgangskomponenten entstehenden Carbonsäurechloride herbeizuführen. Im aus dem Destillationsreaktor austretenden und in den Mitteleinlauf der nachgeschalteten Destillationskolonne eintretenden Reaktionsgemisch soll nach Möglichkeit der Restchlorgehalt 8 000 ppm nicht überschreiten. Darüber geringfügig hinausgehende Chlorgehalte sind jedoch nicht problematisch, da durch eine diesen Gehalten angepaßte Fahrweise der dem Destillationsreaktor nachgeschalteten Kolonne zu hohe Eingangschlorgehalte kompensiert werden können, ohne daß geringste Qualitätsminderungen in den die Kolonnenblase verlassenden Produkten festzustellen sind.

Es hat sich gezeigt, daß zur Erzielung wirtschaftlicher Raum-Zeit-Ausbeuten die in den Reaktoren angewandte Temperatur 25 °C nicht unterschreiten sollte. Ebenso wird die Raum-Zeit-Ausbeute gemindert, wenn die in den Reaktoren angewandte Temperatur in einem Temperaturbereich liegt, in dem die intermolekulare Kondensation der Carbonoyloxysilane zu Siloxanen verstärkt einsetzt. Der Temperaturbereich, in dem die intermolekulare Kondensation der Carbonoyloxysilane einsetzt, hängt sowohl von dem eingesetzten Chlorsilan als auch von der das Chlor im Chlorsilan substituierenden Carbonoyloxy-Gruppierung ab, so daß von Fall zu Fall in Vorversuchen die optimale Reaktionstemperatur ermittelt werden muß. Bei der Synthese der meisten Carbonoyloxysilane mit praktischer Anwendung ergeben sich bezüglich der Raum-Zeit-Ausbeute keine Unterschiede, wenn die in den einzelnen Reaktionseinheiten des Reaktorsystems jeweils angewandte Reaktionstemperatur im Temperaturbereich von 50 bis 90 °C gehalten wird.

Die bisher dargelegte Verfahrensweise der Umsetzung der Ausgangsprodukte Organo-Chlorsilan und Monocarbonsäureanhydrid in den Reaktionseinheiten des Reaktorsystems ist nur mit wirtschaftlich akzeptablen Raum-Zeit-Ausbeuten zu praktizieren, wenn entsprechend der vorliegenden Erfindung die Umsetzung der eingespeisten Ausgangsstoffe in Gegenwart katalytisch wirkender Zusätze erfolgt.

Als katalytisch wirksame Substanzen zur Durchführung der erfindungsgemäßen Verfahrensweise haben sich organische Basen, deren Salze und Säureamide als besonders geeignet erwiesen.

So können als organische Basen primäre, sekundäre und tertiäre Amine eingesetzt werden. Beispielhaft seien die Verbindungen

Phenylamin, Cyclohexylamin,

Propylamin, Iso-Propylamin,

Morpholin, Piperidin,

Dicyclohexylamin, Triethylamin,
Triethanolamin, Pyridin,
Benzothiazol und Methylpyrrolidon

genannt. Es wurde gefunden, daß im Sinne der vorliegenden Erfindung auch sekundäre und tertiäre Amine mit verschiedenen Gruppen im Molekül, z. B. Methylethylamin bzw. Dimethylethylamin, wirksam sind. Gemische der genannten Amine zeigen ebenfalls entsprechende Wirksamkeit, ohne daß dabei positive oder negative synergistische Effekte gefunden wurden.

Bezüglich der Wirksamkeit bei der Durchführung der erfindungsgemäßen Umsetzungen haben sich keine Unterschiede bei Verwendung der Salze gegenüber dem Einsatz der Basen selbst gezeigt. Die bevorzugt eingesetzte Salzform ist das Hydrochlorid. Aber auch die Hydrobromide und die mit organischen Säuren gebildeten Salze, wie z. B. die Propionate, sind ohne Wirksamkeitseinschränkung einsetzbar. Die Zugabe der Salze der eingesetzten organischen Basen zu den Reaktions-Ausgangsstoffen erfolgt bevorzugt in gelöster Form, wobei als Lösungsmittel z. B. Ethanol geeignet ist.

Als gleich wirksam wie die organischen Basen und deren Salze haben sich zur Beschleunigung des Reaktionsablaufes bei der erfindungsgemäßen Umsetzung organische Säureamide erwiesen. Beispielhaft seien die Verbindungen

Acetamid, N-Methylacetamid,
N,N-Methylethylpropionamid,
Benzoesäureamid und Thioharnstoff

aufgeführt. Die Zugabe der Säureamide erfolgt in flüssiger, ggf. auch in gelöster Form. Auch Gemische obiger Säureamide zeigen entsprechende Wirksamkeit, ohne daß dabei synergistische Effekte beobachtet werden.

Die Menge der anzuwendenden Zusätze kann in weiten Bereichen variiert werden. Zur Erzielung optimaler Wirkungen können die dem Gemisch der Einsatzstoffe zuzusetzenden Mengen an wirksamer Substanz von 5 bis 1 000 ppm betragen. Der bevorzugte Bereich liegt bei 10 bis 100 ppm, bezogen auf das jeweilige Gemisch der zur Umsetzung vorgesehenen Reaktanten. Der Einsatz größerer Mengen bringt keine weiteren Vorteile.

Aus dem Destillationsreaktor gelangt das im wesentlichen von den entsprechenden Carbonsäurechloriden befreite Reaktionsgemisch in den Mitteleinlauf einer Kolonne, die in an sich bekannter Weise als Destillationskolonne benutzt wird. Die Kolonne kann sowohl bei Normaldruck als auch bei Unterdruck betrieben werden. Die Arbeitsweise der Kolonne wird der jeweiligen thermischen Stabilität der hergestellten Carbonoyloxysilane angepaßt. Die Entfernung des Carbonsäurechlorids aus dem Destillationsreaktor erfolgt bevorzugt bei einem Druck von 5 bis 300 mbar. Die besonders bevorzugte Betriebsweise besteht darin, die Kolonne bei einem Druck von 5 bis 25 mbar zu betreiben. Bei dieser Betriebsweise stellt sich als Kolonnenkopftemperatur der dem jeweiligen Kolonneninnendruck entsprechende Siedebereich des eingesetzten Carbonsäureanhydrids ein.

Wie bereits erwähnt, wird die Arbeitsweise der Kolonne der thermischen Stabilität der jeweils gebildeten Carbonoyloxysilane angepaßt, das heißt, der Arbeitsdruck in der Kolonne wird so gewählt, daß in der Kolonnenblase eine Temperatur herrscht, die unterhalb der Temperatur liegt, bei der die intermolekulare Kondensation des vorliegenden Carbonoyloxysilans merklich einsetzt.

Bevorzugt werden je Grammatom silicium-gebundenen Chlors 1,05 bis 1,2 Mol Carbonsäureanhydrid eingesetzt.

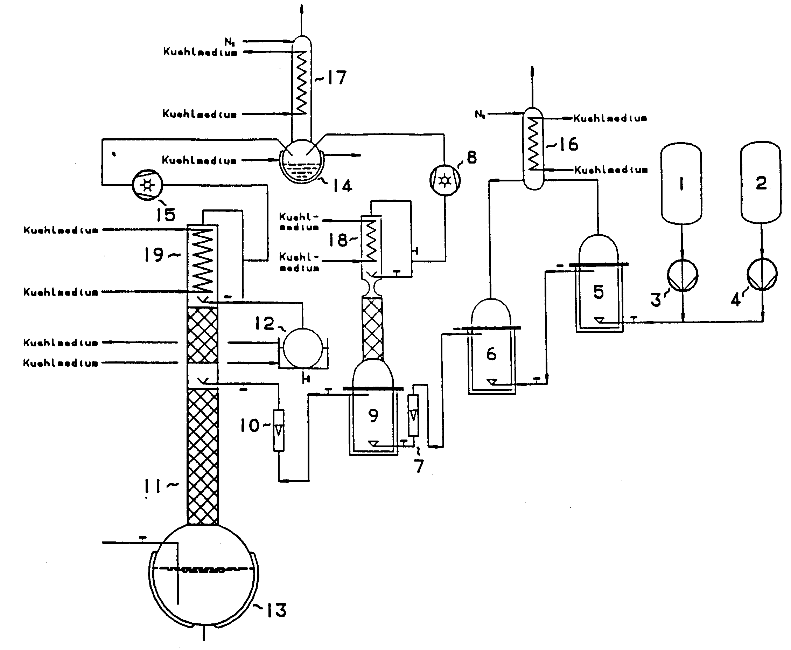

Die Erfindung wird nachfolgend anhand der Zeichnung und der Beispiele erläutert.

Die Zeichnung zeigt schematisch eine Ausführungsform einer Anlage zur Durchführung des erfindungsgemäßen Verfahrens. Die Einsatzstoffe Organo-Chlorsilan bzw. Carbonsäureanhydrid mit katalytisch wirkenden Zusatzstoffen werden aus den Vorlagebehältern 1 bzw. 2 über die Dosierpumpen 3 und 4 kontinuierlich im festgelegten Mengenverhältnis in den Reaktor 5 eingespeist und dort auf Reaktionstemperatur gebracht. Aus dem Reaktor 5 gelangt das Reaktionsgemisch in den Reaktor 6, von dem aus es über den Rotamesser 7 dosiert in den an ein Vakuumsystem 8 angeschlossenen Destillationsreaktor 9 geleitet wird. In diesem wird aus dem Reaktionsgemisch das gebildete Carbonoylchlorid entfernt und nach Passieren der Vakuumpumpe 8 in der Kühlvorlage 14 gesammelt. Das verbleibende Produkt, das noch geringe Anteile Carbonsäurechlorid und unumgesetzte Ausgangsprodukte enthalten kann, wird über den Mengendurchflußmesser 10 in den Mitteleinlauf der mit Sattelkörpern befüllten Säule 11 einer Destillationskolonne, die an ein Vakuumsystem 15 angeschlossen ist, eingespeist. Überschüssiges Carbonsäureanhydrid wird im oberen Teil der Kolonne abgenommen und kontinuierlich der gekühlten Vorlage 12 zugeführt. Das Carbonoyloxysilan wird kontinuierlich aus der Kolonnenblase 13 abgezogen.

Beispiel 1 (Vergleichsbeispiel)

Herstellung von Ethyl-tris(ethanoyloxy)silan und Ethanoylchlorid

Ethyltrichlorsilan wird in den Vorlagebehälter 1, Ethansäureanhydrid in die Vorlage 2 gefüllt. Mittels der Dosierpumpen 3 bzw. 4 werden 125 g (0,765 mol) Ethyltrichlorsilan pro Stunde und 281 g (2,76 mol) Ethansäureanhydrid pro Stunde in den unteren Teil des Reaktors 5 (Vol. = 1 Lit.) eingespeist, in dem die Erwärmung der Einsatzstoffe auf 60 $^{\circ}$C erfolgt. Nach Durchlaufen des Reaktors 5 gelangt das Reaktionsgemisch über den Kühler 16 in den Reaktor 6 (Vol. =

1 Lit.), in dem es auf 60 °C gehalten wird und aus dem es anschließend über den Rotamesser 7 in einer Menge von ca. 406 g pro Stunde in den Destillationsreaktor 9 (Vol. = 1 Lit.) eingegeben wird. In diesem wird das Reaktionsgemisch auf 90 °C erwärmt und das bisher gebildete und sich noch bildende Ethanoylchlorid bei einem Druck von 50 bis 60 mbar (Vakuumpumpe 8) aus dem Reaktionsgemisch abdestilliert und über den Kühler 18 in die gekühlte Vorlage 14 abgeführt. Die pro Stunde aufgefangene Menge Ethanoylchlorid in der Vorlage 14 beträgt ca. 172 g (2,2 mol).

Über den Rotamesser 10 wird das im Oberteil des Destillationsreaktors 9 abgezogene Produkt in den Mitteleinlauf der Destillationssäule 11 der Kolonne aufgegeben, die aus einem 1,60 m langen Glasrohr mit einem Durchmesser von 5 cm besteht und mit Sattelkörpern von 6 mm Durchmesser gefüllt ist. Der Kühler 19 des Kolonnenkopfes Und die Destillatvorlage 12 werden mit einer Kühlflüssigkeit (- 27 °C) beaufschlagt. Den unteren Abschluß der Kolonne bildet ein 4-1-Doppelmantelkolben 13, beheizt durch einen Thermostaten (Umlauftemperatur etwa 125 °C). Bei einem Kolonneninnendruck von 5 bis 7 mbar und einer Temperatur von ca. 110 °C in der Kolonnenblase 13 wird das eingespeiste Rohprodukt aufgearbeitet. Aus dem zur Hälfte mit Flüssigkeit befüllten Doppelmantelkolben 13 wird Ethyl-tris(ethanoyloxy)silan ständig in einem Maß abgenommen, daß der Flüssigkeitsspiegel im Kolben 13 unverändert bleibt. Im oberen Teil der Kolonne destilliert überschüssiges Ethansäureanhydrid ab, welches in der Destillatvorlage 12 gesammelt wird. Am Kopf der Kolonne wird über den Kühler 19 und die Vakuumpumpe 15 das restliche Ethanoylchlorid in die mit dem Kühler 17 versehene Vorlage 14 abdestilliert.

Das dem Doppelmantelkolben 13 entnommene Produkt besteht aus

| | |
|---|---|
| Ethyl-tris(ethanoyloxy)silan | ca. 95,8 Gew.-% |
| Ethyl-di(ethanoyloxy)chlorsilan | ca. 0,37 Gew.-% |
| Ethyl-ethanoyloxy-dichlorsilan | ca. 0,06 Gew.-% |
| Siloxane | ca. 1,9 Gew.-% |
| Ethansäureanhydrid | ca. 1,8 Gew.-% |
| Chlor, hydrolysierbar | ca. 800 ppm. |

Ausbeute: 97,5 %.

Beispiel 2 (Vergleichsbeispiel)

Herstellung von Methyl-tris(propanoyloxy)silan und Propanoylchlorid

Die in Beispiel 1 beschriebene Arbeitsweise wird wiederholt unter Vornahme folgender Veränderungen:
Anstelle von Ethyltrichlorsilan und Ethausäureanhydrid werden Methyltrichlorsilan und Propansäureanhydrid zur Umsetzung gebracht. Methyltrichlorsilan wird in einer Menge von 114 g (0,765 mol) pro Stunde, Propansäureanhydrid in einer Menge von 383 g (2,94 mol) pro Stunde in das Reaktorsystem eingespeist. Die pro Stunde in der Vorlage 14 aufgefangene Menge an Propanoylchlorid beträgt ca. 212 g (2,29 mol). Das aus dem Doppelmantelkolben 13 der Destillationzkolonne abgezogene Produkt setzt sich wie folgt zusammen:

| | |
|---|---|
| Methyl-tris(propanoyloxy)silan | ca. 96,1 Gew.-% |
| Methyl-bis(propanoyloxy)chlorsilan | ca. 0,32 Gew.-% |
| Methyl-propanoyloxydichlorsilan | ca. 0,05 Gew.-% |
| Siloxane | ca. 1,7 Gew.-% |
| Propansäureanhydrid | ca. 1,8 Gew.-% |
| Chlor, hydrolysierbar | 700 ppm. |

Ausbeute: 96,9 %.

Beispiel 3 (Vergleichsbeispiel)

Herstellung von Methylpropyl-bis(ethanoyloxy)silan und Ethanoylchlorid

Die in Beispiel 1 beschriebene Arbeitsweise wird wiederholt unter Vornahme nachfolgender Änderungen: Anstelle von Ethyltrichlorsilan wird Methylpropyldichlorsilan mit Ethansäureanhydrid zur Umsetzung gebracht. Methylpropyldichlorsilan wird in einer Menge von 120 g (0,765 mol) pro Stunde, Ethansäureanhydrid in einer Menge von 234 g (2,29 mol) pro Stunde in das Reaktorsystem eingespeist.

Die pro Stunde in der Vorlage 14 aufgefangene Menge an Ethanoylchlorid beträgt ca. 120 g (1,53 mol).

Das aus dem Doppelmantelkolben 13 der Kolonne abgezogene Produkt setzt sich wie folgt zusammen:

| Methylpropyl-bis(ethanoyloxy)silan | 96,7 Gew.-% |
|---|---|
| Methylpropyl-ethanoyloxy-chlorsilan | 0,7 Gew.-% |
| Siloxane | 1,5 Gew.-% |
| Ethansäureanhydrid | 1,1 Gew.-% |
| Chlor, hydrolysierbar | ca. 1 180 ppm. |

Ausbeute: 97,5 %.

Beispiel 4

Herstellung von Ethyl-tris(ethanoyloxy)silan und Ethanoylchlorid

Die in Beispiel 1 beschriebene Arbeitsweise wird wiederholt unter Vornahme nachfolgender Änderungen: Anstelle von 125 g (0,765 mol) Ethyltrichlorsilan und 281 g (2,76 mol) Ethansäureanhydrid gelangen 156 g (0,96 mol) Ethyltrichlorsilan und 352 g (3,46 mol) Ethansäureanhydrid, versetzt mit 51 mg Triethylamin (entsprechend 100 ppm, bezogen auf das Gemisch der Reaktanten), pro Stunde zur Einspeisung.

Das dem Doppelmantelkolben 13 entnommene Produkt besteht aus:

| Ethyl-tris(ethanoyloxy)silan | 97,2 Gew.-% |
|---|---|
| Ethyl-di(ethanoyloxy)chlorsilan | - |
| Ethyl-ethanoyloxy-dichlorsilan | - |
| Siloxane | 1,7 Gew.-% |
| Ethansäureanhydrid | 1,1 Gew.-% |
| Chlor, hydrolysierbar | 3 ppm. |

Ausbeute: 97,8 %.

Beispiel 5

Herstellung von Ethyl-tris(ethanoyloxy)silan und Ethanoylchlorid

Die in Beispiel 4 beschriebene Arbeitsweise wird wiederholt. Anstelle von 51 mg Triethylamin wird dem Ethansäureanhydrid eine Menge von 5,1 mg Triethylamin (entsprechend 10 ppm, bezogen auf das Gemisch der Reaktanten) zugesetzt.

Das dem Doppelmantelkolben 13 entnommene Produkt besteht aus:

| | |
|---|---|
| Ethyl-tris(ethanoyloxy)silan | 97,2 Gew.-% |
| Ethyl-di(ethanoyloxy)chlorsilan | - |
| Ethyl-ethanoyloxy-dichlorsilan | - |
| Siloxane | 1,9 Gew.-% |
| Ethansäureanhydrid | 0,9 Gew.-% |
| Chlor, hydrolysierbar | 5 ppm. |

Ausbeute: 97,6 %.

Beispiele 6 bis 9

Herstellung von Ethyl-tris(ethanoyloxy)silan und Ethanoylchlorid

Die in Beispiel 4 beschriebene Arbeitsweise wird wiederholt. Anstelle von 51 mg Triethylamin wird dem Ethansäureanhydrid eine Menge von
  25 mg Phenylamin oder
  25 mg Cyclohexylamin oder
  25 mg n-Propylamin oder
  25 mg iso-Propylamin
(entsprechend 50 ppm, bezogen auf das Gemisch der Reaktanten) zugesetzt.
  Die dem Doppelmantelkolben 13 entnommenen Produkte weisen folgenden Zusammensetzungsbereich auf:

| | |
|---|---|
| Ethyl-tris(ethanoyloxy)silan | 97,2 bis 97,4 Gew.-% |
| Ethyl-di(ethanoyloxy)chlorsilan | - |
| Ethyl-ethanoyloxy-dichlorsilan | - |
| Siloxane | 1,9 bis 2,0 Gew.-% |
| Ethansäureanhydrid | 0,6 bis 0,9 Gew.-% |
| Chlor, hydrolysierbar | 2 bis 7 ppm. |

Ausbeute: 97,2 bis 97,4 %.

Beispiele 10 bis 12

Herstellung von Ethyl-tris(ethanoyloxy)silan und Ethanoylchlorid

Die in Beispiel 4 beschriebene Arbeitsweise wird wiederholt. Anstelle von 51 mg Triethylamin wird dem Ethansäureanhydrid eine Menge von
  18 mg Diethylamin oder
  18 mg Dicyclohexylamin oder
  18 mg Piperidin
(entsprechend 35 ppm, bezogen auf das Gemisch der Reaktanten) zugesetzt.

Die dem Doppelmantelkolben 13 entnommenen Produkte weisen folgenden Zusammensetzungsbereich auf:

| | |
|---|---|
| Ethyl-tris(ethanoyloxy)silan | 97,0 bis 97,3 Gew.-% |
| Ethyl-di(ethanoyloxy)chlorsilan | - |
| Ethyl-ethanoyloxy-dichlorsilan | - |
| Siloxane | 1,6 bis 2,3 Gew.-% |
| Ethansäureanhydrid | 0,7 bis 1,1 Gew.-% |
| Chlor, hydrolysierbar | 2 bis 5 ppm. |

Ausbeute: 97,1 bis 97,9 %.

Beispiele 13 bis 15

Herstellung von Ethyl-tris(ethanoyloxy)silan und Ethanoylchlorid

Die in Beispiel 4 beschriebene Arbeitsweise wird wiederholt. Anstelle von 51 mg Triethylamin wird dem Ethansäureanhydrid eine Menge von
     34 mg Pyridin oder
     34 mg Benzothiazol oder
     34 mg Methylpyrrolidon
(entsprechend 65 ppm, bezogen auf das Gemisch der Reaktanten) zugesetzt.
Die dem Doppelmantelkolben 13 entnommenen Produkte weisen folgenden Zusammensetzungsbereich auf:

| | |
|---|---|
| Ethyl-tris(ethanoyloxy)silan | 96,5 bis 97,4 Gew.-% |
| Ethyl-di(ethanoyloxy)chlorsilan | - |
| Ethyl-ethanoyloxy-dichlorsilan | - |
| Siloxane | 2,1 bis 2,4 Gew.-% |
| Ethansäureanhydrid | 0,7 bis 1,1 Gew.-% |
| Chlor, hydrolysierbar | 3 bis 7 ppm. |

Ausbeute: 96,8 bis 97,4 %.

Beispiel 16

Herstellung von Ethyl-tris(ethanoyloxy)silan und Ethanoylchlorid

Die in Beispiel 4 beschriebene Arbeitsweise wird wiederholt. Anstelle von 51 mg Triethylamin wird dem Ethansäureanhydrid eine Menge von 52 mg Triethylamin-Hydrochlorid (gelöst in Ethanol, entsprechend 50 ppm, bezogen auf das Gemisch der Reaktanten) zugesetzt.

Das dem Doppelmantelkolben 13 entnommene Produkt weist folgende Zusammensetzung auf:

| Ethyl-tris(ethanoyloxy)silan | 97,6 Gew.-% |
|---|---|
| Ethyl-di(ethanoyloxy)chlorsilan | - |
| Ethyl-ethanoyloxy-dichlorsilan | - |
| Siloxane | 1,6 Gew.-% |
| Ethansäureanhydrid | 0,8 Gew.-% |
| Chlor, hydrolysierbar | 3 ppm. |

Ausbeute: 97,9 %.

Beispiele 17 bis 19

Herstellung von Ethyl-tris(ethanoyloxy)silan und Ethanoylchlorid

Die in Beispiel 4 beschriebene Arbeitsweise wird wiederholt. Anstelle von 51 mg Triethylamin wird dem Ethansäureanhydrid eine Menge von
50 mg Acetamid (gelöst in Ethanol) oder
25 mg N,N-Methylethylacetamid oder
12 mg Thioharnstoff (gelöst in Ethansäure)
(entsprechend 100 ppm bzw. 50 ppm bzw. 25 ppm, bezogen auf das Gemisch der Reaktanten) zugesetzt.
Die dem Doppelmantelkolben 13 entnommenen Produkte weisen folgenden Zusammensetzungsbereich auf:

| Ethyl-tris(ethanoyloxy)silan | 97,0 bis 97,2 Gew.-% |
|---|---|
| Ethyl-di(ethanoyloxy)chlorsilan | - |
| Ethyl-ethanoyloxy-dichlorsilan | - |
| Siloxane | 1,6 bis 2,1 Gew.-% |
| Ethansäureanhydrid | 0,9 bis 1,2 Gew.-% |
| Chlor, hydrolysierbar | 5 ppm. |

Ausbeute: 97,4 bis 97,8 %.

Beispiele 20 bis 24

Herstellung von Methylpropyl-bis(ethanoyloxy)silan und Ethanoylchlorid

Die in Beispiel 3 beschriebene Arbeitsweise wird wiederholt unter Vornahme folgender Änderungen:
Anstelle von 120 g (0,765 mol) Methylpropyl-dichlorsilan und 234 g (2,29 mol) Ethansäureanhydrid werden 150 g (0,96 mol) Methylpropyldichlorsilan und 230 g (2,25 mol) Ethansäureanhydrid, dem eine Menge von
30 mg n-Propylamin oder
30 mg Diethylamin oder
30 mg Benzothiazol oder
30 mg Triethylamin-Hydrochlorid (gelöst in Ethanol) oder
30 mg Acetamid
zugesetzt wird (entsprechend 75 ppm, bezogen auf das Gemisch der Reaktanten), pro Stunde in das Reaktorsystem eingespeist. Die dem Doppelmantelkolben 13 entnommenen Produkte weisen folgenden Zusammensetzungsbereich auf:

| Methylpropyl-bis(ethanoyloxy)silan | 97,1 bis 97,4 Gew.-% |
|---|---|
| Methylpropyl-ethanoyloxy-chlorsilan | - |
| Siloxane | 1,6 bis 2,2 Gew.-% |
| Ethansäureanhydrid | 0,7 bis 1,0 Gew.-% |
| Chlor, hydrolysierbar | 5 bis 7 ppm. |

Ausbeute: 97,0 bis 97,9 %.

Beispiel 25

Herstellung von Vinyl-tris(propanoyloxy)silan und Propanoylchlorid

Die in Beispiel 2 beschriebene Arbeitsweise wird wiederholt unter Vornahme folgender Abänderungen: Anstelle von 114 g (0,765 mol) Methyltrichlorsilan und 383 g (2,94 mol) Propansäureanhydrid werden 156 g (0,956 mol) Vinyltrichlorsilan und 440 g (3,38 mol) Propansäureanhydrid, versetzt mit 30 mg Methylethylamin und 30 mg Ethylamin, pro Stunde in das Reaktorsystem eingespeist.

Das dem Doppelmantelkolben 13 entnommene Produkt setzt sich wie folgt zusammen:

| Vinyl-tris(propanoyloxy)silan | 97,3 Gew.-% |
|---|---|
| Vinyl-bis(propanoyloxy)chlorsilan | - |
| Vinyl-propanoyloxydichlorsilan | - |
| Siloxane | 1,8 Gew.-% |
| Propansäureanhydrid | 0,9 Gew.-% |
| Chlor, hydrolysierbar | 9 ppm. |

Ausbeute: 97,7 %.

Beispiel 26

Herstellung von 2-Chlorethylmethyl-bis(ethanoyloxy)silan und Ethanoylchlorid

Die in Beispiel 4 beschriebene Arbeitsweise wird wiederholt. Anstelle von 156 g (0,96 mol) Ethyltrichlorsilan und 352 g (3,46 mol) Ethansäureanhydrid, versetzt mit 51 mg Triethylamin, gelangen 255 g (1,44 mol) 2-Chlorethylmethyldichlorsilan und 346 g (3,40 mol) Ethansäureanhydrid, versetzt mit 51 mg Triethylamin, pro Stunde zur Einspeisung in die Reaktoren. Das aus dem Doppelmantelkolben 13 entnommene Produkt weist folgende Zusammensetzung auf:

| 2-Chlorethylmethyl-bis(ethanoyloxy)silan | 97,2 Gew.-% |
|---|---|
| 2-Chlorethylmethyl-ethanoyloxy-chlorsilan | - |
| Siloxane | 1,8 Gew.-% |
| Ethansäureanhydrid | 1,0 Gew.-% |
| Chlor, hydrolysierbar | 10 ppm. |

Ausbeute: 97,6 %.

**Patentansprüche**

1. Verfahren zur gleichzeitigen und kontinuierlichen Herstellung von Carbonoyloxysilanen und Carbonsäurechloriden durch Umsetzung von Organo-Chlorsilanen mit überschüssigem Monocarbonsäureanhydrid bei erhöhter Temperatur, Abtrennung des Carbonsäurechlorids nach erfolgter Umsetzung und daran anschließender Gewinnung des Carbonoyloxysilans, dadurch gekennzeichnet, daß man die Ausgangskomponenten unter Zusatz von im Reaktionsgemisch löslichen organischen Basen, deren Salzen oder organischen Säureamiden bei Temperaturen von 25 bis 100 °C durch einen Reaktor oder mehrere Reaktoren leitet, anschließend in einem Destillationsreaktor das entstandene Carbonsäurechlorid im Vakuum entfernt und das nach Abtrennung des Carbonsäurechlorids verbleibende Reaktionsgemisch in den Mitteleinlauf einer Kolonne überführt, unter Vakuum am Kolonnenkopf überschüssiges Carbonsäureanhydrid abdestilliert und das Carbonoyloxysilan aus der Kolonnenblase abzieht.

2. Verfahren nach Anspruch 1, dadurch gekennzeichnet, daß als organische Basen tertiäre, sekundäre oder primäre Amine zugesetzt werden.

3. Verfahren nach Anspruch 1, dadurch gekennzeichnet, daß die Salze tertiärer, sekundärer oder primärer Amine zugesetzt werden.

4. Verfahren nach Anspruch 1, dadurch gekennzeichnet, daß nicht-substituierte und/oder N-substituierte Säureamide zugesetzt werden.

5. Verfahren nach Anspruch 2, dadurch gekennzeichnet, daß Di- und/oder Trialkylamine zugesetzt werden.

6. Verfahren nach Anspruch 3, dadurch gekennzeichnet, daß Di- und/oder Trialkylamin-Hydrochloride zugesetzt werden.

7. Verfahren nach Anspruch 4, dadurch gekennzeichnet, daß mono- oder di-alkylsubstituierte Carbonsäureamide zugesetzt werden.

8. Verfahren nach einem der Ansprüche 1 bis 7, dadurch gekennzeichnet, daß die organischen Basen, deren Salze oder die organischen Säureamide in Mengen von 5 bis 1 000 ppm, bezogen auf das Gemisch der Ausgangsstoffe, eingesetzt werden.

9. Verfahren nach Anspruch 8, dadurch gekennzeichnet, daß die organischen Basen, deren Salze oder die organischen Säureamide in Mengen von 10 bis 100 ppm, bezogen auf das Gemisch der Ausgangsstoffe, eingesetzt werden.

10. Verfahren nach einem der Ansprüche 1 bis 9, dadurch gekennzeichnet, daß die Obergrenze der Reaktionstemperatur in den Reaktoren unterhalb der Temperatur liegt, bei der die intermolekulare Kondensation des gebildeten Carbonoyloxysilans merklich einsetzt.

11. Verfahren nach Anspruch 10, dadurch gekennzeichnet, daß die Umsetzungstemperatur in den Reaktoren 50 bis 90 °C beträgt.

12. Verfahren nach einem der Ansprüche 1 bis 11, dadurch gekennzeichnet, daß je Grammatom silicium-gebundenen Chlors 1,05 bis 1,2 Mol Carbonsäureanhydrid eingesetzt werden.

13. Verfahren nach einem der Ansprüche 1 bis 12, dadurch gekennzeichnet, daß die Entfernung des Carbonsäurechlorids aus dem Destillationsreaktor bei einem Druck von 5 bis 300 mbar erfolgt.

**Claims**

1. A process for the simultaneous and continuous preparation of carbonoyloxysilanes and carboxylic acid chlorides by reaction of organochlorosilanes with excess monocarboxylic acid anhydride at elevated temperature, separation of the carboxylic acid chloride after the reaction has taken place, and subsequent obtainment of the carbonoyloxysilane, characterised in that the starting components with the addition of organic bases soluble in the reaction mixture, the salts thereof or organic acid amides are passed through one or more reactors at temperatures of 25 to 100°C, the resulting carboxylic acid chloride is subsequently removed under vacuum in a distillation reactor, and the reaction mixture remaining after separation of the carboxylic acid chloride is transferred to the central inlet of a column, excess carboxylic acid anhydride is distilled off under vacuum at the column head and the carbonoyloxysilane is drawn off from the bulb of the column.

2. A process according to claim 1, characterised in that the organic bases added are tertiary, secondary or primary amines.

3. A process according to claim 1, characterised in that the salts of tertiary, secondary or primary amines are added.

4. A process according to claim 1, characterised in that unsubstituted and/or N-substituted acid amides are added.

5. A process according to claim 2, characterised in that di- and/or trialkylamines are added.

6. A process according to claim 3, characterised in that di- and/or trialkylamine hydrochlorides are added.

7. A process according to claim 4, characterised in that mono- or dialkylsubstituted carboxylic acid amides are added.

8. A process according to one of claims 1 to 7, characterized in that the organic bases, the salts thereof or the organic acid amides are used in quantities of 5 to 1,000 ppm, based on the mixture of starting materials.

9. A process according to claim 8, characterized in that the organic bases, the salts thereof or the organic acid amides are used in quantities of 10 to 100 ppm, based on the mixture of starting materials.

10. A process according to one of claims 1 to 9, characterized in that the upper limit of the reaction temperature in the reactors is below the temperature at which intermolecular condensation of the carbonoyloxysilane formed commences to an appreciable degree.

11. A process according to claim 10, characterised in that the reaction temperature in the reactors is 50 to 90°C.

12. A process according to one of claims 1 to 11, characterised in that 1.05 to 1.2 mol of carboxylic acid anhydride are used per gram-atom of silicon-bound chlorine.

13. A process according to one of claims 1 to 12, characterised in that the removal of the carboxylic acid chloride from the distillation reactor takes place at a pressure of 5 to 300 mbar.

## Revendications

1. Procédé de préparation simultanée et en continu de carbonoyloxysilanes et de chlorures d'acide carboxylique par réaction d'organochorosilanes avec un anhydride d'acide monocarboxylique en excès, à température élevée, par séparation du chlorure d'acide carboxylique après achèvement de la réaction et obtention finale du carbonoyloxysilane, caractérisé en ce que l'on conduit les composants de départ tout en ajoutant des bases organiques solubles dans le mélange réactionnel, leurs sels ou leurs amides d'acide organiques, à des températures allant de 25 à 100°C, à travers un ou plusieurs réacteurs, on élimine ensuite dans un réacteur de distillation le chlorure d'acide carboxylique sous vide, en ce que l'on transfère après séparation du chlorure d'acide carboxylique le mélange réactionnel à une entrée médiane d'une colonne, que l'on sépare par distillation sous vide, en tête de la colonne, l'anhydride d'acide carboxylique en excès et que l'on soutire le carbonoyloxysilane de la colonne.

2. Procédé selon la revendication 1, caractérisé en ce que l'on ajoute comme bases organiques, des amines tertiaires, secondaires ou primaires.

3. Procédé selon la revendication 1, caractérisé en ce que l'on ajoute les sels d'amine tertiaire, secondaire ou primaire.

4. Procédé selon la revendication 1, caractérisé en ce que l'on ajoute des amides d'acide non substitués et/ou N-substitués.

5. Procédé selon la revendication 2, caractérisé en ce que l'on ajoute des di-et/ou des trialcoylamines.

6. Procédé selon la revendication 3, caractérisé en ce que l'on ajoute les chlorhydrates de di-et/ou trialcoylamines.

**7.** Procédé selon la revendication 4, caractérisé en ce que l'on ajoute des amides d'acide carboxylique mono-ou di-substituées par un alcoyle.

**8.** Procédé selon l'une des revendications 1 à 7, caractérisé en ce que l'on met en jeu les bases organiques, leurs sels ou les amides d'acide carboxylique, organiques en quantités allant de 5 à 1000 ppm rapporté au mélange des produits de départ.

**9.** Procédé selon la revendication 8, caractérisé en ce que l'on met en jeu les bases organiques, leurs sels ou les amides d'acide carboxylique organiques en quantités allant de 10 à 100 ppm rapporté au mélange des produits de départ.

**10.** Procédé selon l'une des revendications 1 à 9, caractérisé en ce que la limite supérieure de la température de réaction dans les réacteurs se situe en dessous de la température à laquelle la condensation intramoléculaire du carbonoyloxysilane formé, commence notablement.

**11.** Procédé selon la revendication 10, caractérisé en ce que la température de réaction dans les réacteurs s'élève à 50 à 90°C.

**12.** Procédé selon l'une des revendications 1 à 11, caractérisé en ce que l'on met en jeu 1,05 à 1,2 mol d'anhydride d'acide carboxylique par atome gramme de chlore lié au silicium.

**13.** Procédé selon l'une des revendications 1 à 12, caractérisé en ce que l'élimination du chlorure d'acide carboxylique du réacteur de distillation, s'effectue à une pression de 5 à 300 mbars.